# EUROPEAN PATENT SPECIFICATION

(11) **EP 2 533 734 B1**
(45) Date of publication and mention of the grant of the patent: **16.01.2019**
(21) Application number: 11741791.5
(22) Date of filing: 02.02.2011
(51) Int. Cl.: A61F 5/01

(54) **ORTHOTIC FOOT BRACE FOR REMEDIATION OF FOOT DROP SYMPTOMS**
FUSSKLAMMERORTHESE ZUR LINDERUNG VON SENKFUSSSYMPTOMEN
ORTHÈSE DE PIED POUR REMÉDIER AUX SYMPTÔMES DU PIED TOMBANT

(30) Priority: 09.02.2010 CA 2692534; 09.02.2010 US 702967
(43) Date of publication of application: 19.12.2012
(73) Proprietor: Ortheses Turbomed Inc. / Turbomed Orthotics Inc., Québec, Québec G1N 2E5 (CA)
(72) Inventor: SAVARD, Stéphane, Québec, Québec G1X 4V3 (CA); CÔTÉ, François, Saint-Etienne-de-Lauzon, Québec G6J 1G2 (CA)
(74) Representative: Delorme, Nicolas
(86) International application number: PCT/CA2011/050060
(87) International publication number: WO 2011/097723

(56) References cited:
- FR-A1- 2 832 923
- GB-A- 117 877
- GB-A- 121 322
- US-A- 2 663 294
- US-A- 3 859 991
- US-B1- 6 299 587
- US-B2- 7 125 392

## Description

### Technical Field of the Invention

The technical field relates to orthotics and, more particularly, to an orthosis for remediation of foot drop symptoms.

### Background

Foot drop, drop foot, and foot dangle are terms which have been employed to describe ankle and toe dorsiflexor paresis resulting in the inability to raise the foot at the ankle, such that the foot inclines towards and scrapes the ground when walking. Dorsiflexion is the motion the ankle joint makes when the foot points upward. This motion needs to occur when the foot comes off the ground so that the toes do not drag.

Foot drop makes walking difficult as the toes tend to drag on the ground which leads to tripping and instability. Patients adapt to this by using their hip muscles to exaggerate lifting the foot above the ground (known as a "steppage gait") or by swinging their leg outward so that the foot can clear the ground (known as "circumduction").

A common remediation technique for foot drop involved the employment of an ankle foot orthosis, or brace. The goal of bracing is to provide patients with a more normal and comfortable gait. These devices often require professional fitting, which may imply taking of impressions of the affected foot, and customized shoes. Significant delays can occur between the fitting and receipt of the customized orthosis.

Several braces have been developed for foot drop. Short leg fixed braces, for instance US patent No. 5,429,588, fit into the footwear, do not flex at ankle joint, and do not allow plantar flexion nor dorsiflexion, i.e. they do not provide quite as natural of a gait. Dorsiflexion assist short leg braces are similar to short leg fixed braces but with a spring-like hinge that acts to raise the foot, i.e. dorsiflex the ankle when the foot comes off of the ground. It offers the advantage of a more normal gait pattern. Solid ankle braces, with or without posterior stop, also fit inside the footwear and have a hinge that allows normal dorsiflexion. They can or cannot allow plantarflexion, i.e. it can or cannot let the foot point downward. Energy return braces also fit inside the footwear and use a natural flex built into the material of the brace to provide assist in dorsiflexion. These braces are often made of polymers or carbon graphite materials.

GB121,322, FR2 832 923 A1 and GB117,877 disclose orthotic foot braces for persons wearing a footwear.

### BRIEF SUMMARY OF THE INVENTION

It is therefore an aim of the present invention to address the above mentioned issues.

According to a general aspect, there is provided an orthotic foot brace for a person wearing a footwear. The orthotic foot brace comprises: a lower leg holder securable around a lower leg of the person; a lower leg strut secured to the lower leg holder and extending downwardly towards the footwear; a foot strut having a rear section secured to the vertical strut, at least one of a median section and a lateral section located on a respective side of the footwear and extending outwardly thereof, and a front section securable to an instep section of the footwear; and at least one brace retaining member secured to the foot strut juxtaposed to the footwear, and having at least a section extending on the median side of the footwear and having at least a section extending on the lateral side of the footwear, outwardly thereof, and restraining a rearwardly oriented pivotal movement of the brace, the at least one brace retaining member having a heel support extending rearwardly of the footwear and being connected to at least one of the lateral section and the median section of the foot strut.

Preferred features of the orthotic foot brace are disclosed in the dependent claims.

### BRIEF DESCRIPTION OF THE DRAWINGS

Fig. 7-25 disclose embodiments of the invention. Fig 1-6 disclose an embodiment of a foot brace useful for the understanding of the invention.
Fig. 1 is a front perspective view of an orthotic foot brace in accordance with a first embodiment, wherein the brace is secured to a person's lower leg and footwear;
Fig. 2 is a front perspective view, exploded, of the orthotic foot brace and footwear shown in Fig. 1;
Fig. 3 is a rear perspective view of the orthotic foot brace shown in Fig. 1, wherein the brace is secured to the person's lower leg and footwear;
Fig. 4 is a side elevation view of the orthotic foot brace shown in Fig. 1, wherein the brace is secured to the footwear;
Fig. 5 is a side elevation view of the orthotic foot brace shown in Fig. 1, wherein the brace is secured to the person's lower leg and footwear;
Fig. 6 is a side elevation view of the orthotic foot brace shown in Fig. 1, wherein the brace is secured to the person's leg and footwear and showing dorsiflexion and plantarflexion movements;
Fig. 7 is a front perspective view of an orthotic foot brace in accordance with a second embodiment, wherein the brace is secured to the person's leg and footwear;
Fig. 8 is a front perspective view, exploded, of the orthotic foot brace and footwear shown in Fig. 7;
Fig. 9 is a rear perspective view of the orthotic foot brace shown in Fig. 7, wherein the brace is secured to the person's leg and footwear;
Fig. 10 is a side elevation view of the orthotic foot brace shown in Fig. 7, wherein the brace is secured to a footwear;
Fig. 11 is a side elevation view of the orthotic foot brace shown in Fig. 7, wherein the brace is secured to the person's leg and footwear;
Fig. 12 is a side elevation view of the orthotic foot brace shown in Fig. 7, wherein the brace is secured to the person's leg and footwear showing dorsiflexion and plantarflexion movements;
Fig. 13 is a front perspective view of an orthotic foot brace in accordance with a third embodiment, wherein the brace is secured to the person's leg and footwear;
Fig. 14 is a front perspective view, exploded, of the orthotic foot brace and footwear shown in Fig. 13;
Fig. 15 is a rear perspective view of the orthotic foot brace shown in Fig. 13, wherein the brace is secured to the person's leg and footwear;
Fig. 16 is a side elevation view of the orthotic foot brace shown in Fig. 13, wherein the brace is secured to the person's leg and footwear;
Fig. 17 is a side elevation view of the orthotic foot brace shown in Fig. 13, wherein the brace is secured to the person's leg and footwear and showing dorsiflexion and plantarflexion movements;
Fig. 18 is a front perspective view of an orthotic foot brace in accordance with a fourth embodiment, wherein the brace is secured to the person's leg and footwear;
Fig. 19 is a front perspective view, exploded, of the orthotic foot brace and footwear shown in Fig. 18;
Fig. 20 is a rear perspective view of the orthotic foot brace shown in Fig. 18, wherein the brace is secured to the person's leg and footwear;
Fig. 21 is a side elevation view of the orthotic foot brace shown in Fig. 18, wherein the brace is secured to the person's leg and footwear;
Fig. 22 is a front perspective view of an orthotic foot brace in accordance with a fifth embodiment, wherein the brace is secured to the person's leg and footwear;
Fig. 23 is a front perspective view, exploded, of the orthotic foot brace and footwear shown in Fig. 22;
Fig. 24 is a rear perspective view of the orthotic foot brace shown in Fig. 22, wherein the brace is secured to the person's leg and footwear; and
Fig. 25 is a side elevation view of the orthotic foot brace shown in Fig. 22, wherein the brace is secured to the person's leg and footwear and showing dorsiflexion and plantarflexion movements.

It will be noted that throughout the appended drawings, like features are identified by like reference numerals.

### DETAILED DESCRIPTION

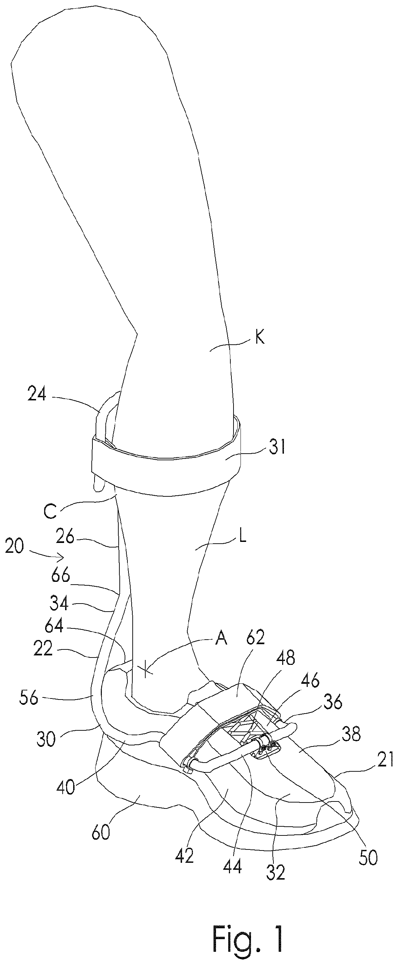

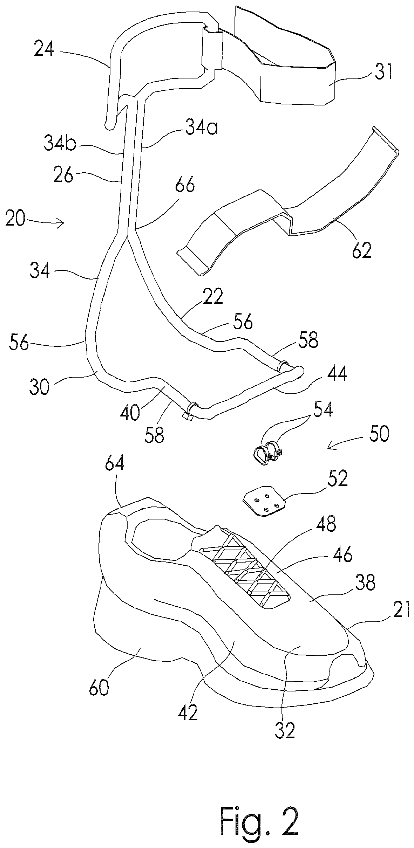

Referring to Figs. 1 to 3, there is shown an orthotic foot brace 20, also referred to ankle-foot orthoses, in accordance with a first embodiment. The foot brace 20 is securable to a person's lower leg L and footwear (or shoe) 21 and encompasses an ankle joint A. It is intended to control position and motion of the person's ankle to compensate for weakness. For conciseness, only the left foot brace which is securable to the person's left lower leg is shown and described below. For this embodiment, the right foot brace is similar or can include only minor modifications.

The brace 20 has a structural frame 22 including a lower leg holder 24 securable to the person's lower leg L, below the knee K, a lower leg vertical section 26 or strut extending from the lower leg holder 24 towards the footwear 21, and a foot section 30 or strut extending from the vertical section 26 towards a toe section 32 of the footwear 21 and securable to the footwear 21, outwardly thereof.

In the embodiment shown, the lower leg holder 24 is a calf cuff juxtaposed to the person's calf C, rearwardly of the person's calf C. More particularly, the calf cuff 24 is designed to abut the upper part of the calf C, below the knee K. A leg attachment strap 31 is secured to one vertical part of the calf cuff 24 and is designed to connect the opposed vertical part of the calf cuff 24 by extending toward the front of the tibia and thereby encircling the person's lower leg L and securing the upper part of the brace 20 thereto. It is appreciated that, in alternative embodiments, the lower leg holder 24 can be positioned at different locations, anywhere above the ankle, below the knee K, and around the lower leg L. For instance and without being limitative, it can be juxtaposed to the person's tibia. It secures the brace 20 to the person's lower leg L. Moreover, it can have a different shape than the one shown in Figs. 1 to 3.

In the embodiment shown, the brace 20 includes a single and continuous frame member 34 extending between the lower leg holder 24, the vertical section 26, and the foot section 30. The frame member 34 includes two frame member sections 34a, 34b juxtaposed in the vertical section 26 and spaced-apart in the lower leg holder 24 and foot section 30. In the embodiment shown, the frame member 34 is a rod with a circular cross-section. However, it is appreciated that it can be a substantially flat member or have any other appropriate shape.

The vertical section 26 extends rearwardly of the person's lower leg L, from the lower leg holder 24 to the foot section 30. It is appreciated that, in alternative embodiments (not shown), it can extend downwardly anywhere around the person's lower leg L. Furthermore, in the embodiment shown, the vertical section 26 has a substantially straight shape. However, in alternative embodiments, it can have a curved or any other appropriate shape. Furthermore, in an alternative embodiment (not shown), the frame member sections 34a, 34b can be spaced-apart from one another either entirely along their length or only along a section thereof. For instance and without being limitative, the frame member sections 34a, 34b can extend downwardly on opposed sides of the lower leg, spaced-apart from one another.

The foot section 30 can be divided into a lateral section 36 which is juxtaposed to a lateral side 38 of the footwear 21, i.e. the outside part of the footwear 21, a median section 40 which is juxtaposed to a median side 42 of the footwear 21, i.e. the inside part of the footwear 21, and a front section 44 which extends above an instep section 46 of the footwear 21, i.e. the part located on the top of the foot, and forwardly of an inner space opening. The frame member 34 extends continuously between each section 36, 40, 44 and is located outwardly of the footwear 21, i.e. it is juxtaposed to the outer surface of the footwear 21.

The brace 20 is secured to the footwear 21 in the front section 44. It is secured to the footwear laces 48 through attachment means 50 including a spreader plate 52 and two attachment members 54. The attachment members 54 attach the spreader plate 52 and the frame member 34 to the laces 48. More particularly, the attachment members 54 are inserted in apertures defined in the spreader plate 52 and surround the footwear laces 48 and the frame member 34. It is appreciated that the brace 20 can be secured to the footwear 21 by any other appropriate technique. For instance and without being limitative, the footwear laces 48 can surround the frame member 34 and fasten the latter. In alternative embodiments (not shown), it can be secured to other footwear components and the attachment means can be adapted in accordance with the footwear design.

In the lateral and median sections 36, 40, the frame member 34 has a substantially curved shaped section 56 to follow the footwear shape in a region corresponding to the person's ankle followed by a substantially straight section 58. The frame member 34 in the lateral and median sections 36, 40 extends longitudinally along the footwear 21, outwardly thereof, above the outsole 60.

A foot band 62 is mounted to the straight sections 58 of the lateral and median sections 36, 40. The foot band 62 encircles the frame member 34 in the lateral and median sections 36, 40 and creates an inwardly directed force. The foot band 62 extends over the instep section 46 of the footwear 21, behind the front section 44 of the frame member 34 and forwardly of the inner space opening. In the embodiment shown, the foot band 62 includes a pair of hook- and-loop type gripping elements, such as "VELCRO®", for securing sections of the foot band 62 together when engaged over the frame member 34 in the lateral and median sections 36, 40. Thus, the tension between the lateral and median sections 36, 40 is adjustable.

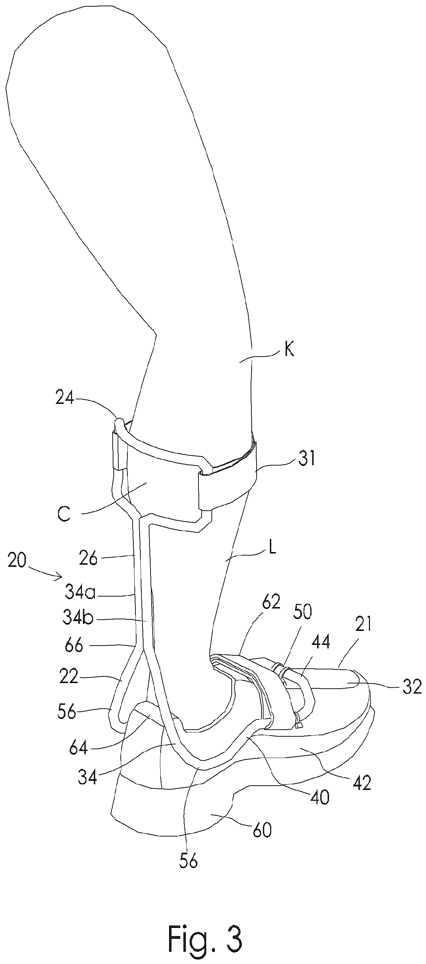

Fig. 3 shows that the frame member sections 34a, 34b are spaced apart above the binding 64 of the footwear 21, i.e. the higher edge of the footwear 21, to allow plantarflexion P, i.e. there is a space defined between the footwear binding 64 and a distal end 66 of the vertical section 26 which corresponds to the meeting point of both frame member sections 34a, 34b. In an alternative embodiment (not shown), the frame member sections 34a, 34b can merge into a single frame member above the meeting point.

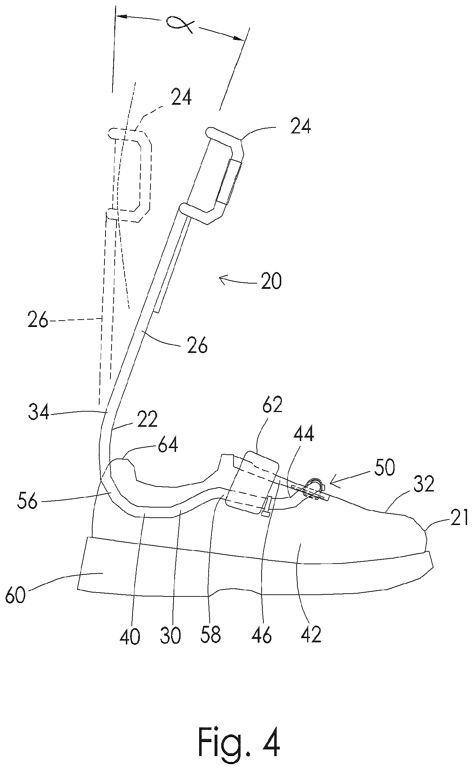

Fig. 4 shows the brace 20 secured to the footwear 21 but without being engaged with a person's lower leg L. The vertical section 26 of the brace 20 and the lower leg holder 24 extend forwardly towards the toe section 32 of the footwear 21. To attach the brace 20 to the lower leg L, the vertical section 26 is pulled rearwardly and, when attached, the lower leg holder 24 and the vertical section 26 apply a forwardly oriented pressure to the lower leg L. In a non-operative configuration, i.e. when detached from the lower leg L, the brace 20 defines an angle α with a vertical axis. The angle ranges between 10 and 30 degrees and, in an alternative embodiment, between 15 and 20 degrees. The compression stress applied to the person's lower leg L restricts the plantarflexion P of the foot and creates a bias for the dorsiflexion D of the foot.

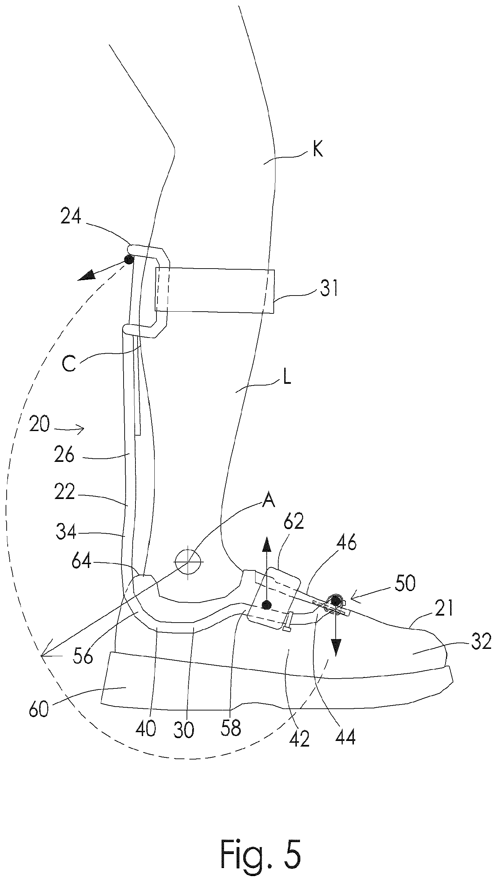

Referring to Figs. 5 to 6, there is shown that during gaiting, the foot including the footwear 21 performs plantarflexions P and pivots about a pivoting axis which substantially corresponds to the ankle joint A. As a result, a tension force is applied on the foot band 62 when the foot performs plantarflexions P and the foot band 62 restricts the plantarflexion P of the foot, i.e. it creates a resistance to the plantarflexion moment P. A rearwardly extending tension is also applied on the lower leg holder 24. As mentioned above, the lower leg holder 24 applies a compression force on the lower leg L. Therefore, the combination of the tension applied to the foot band 62, the rearwardly extending tension, and the compression force applied on the lower leg L creates the resulting dorsiflexion moment D. The brace 20 conveys the foot including the footwear 21 to return to its normal, resting position.

The foot band 62 acts as a retaining member by restraining a rearwardly and downwardly oriented pivotal movement of the brace 20 and, more particularly, by restraining a downward movement of the lower leg holder 24, of the vertical section 26, and/or of the curved sections 56 during gaiting.

The same brace 20 can be used for either the left or the right foot.

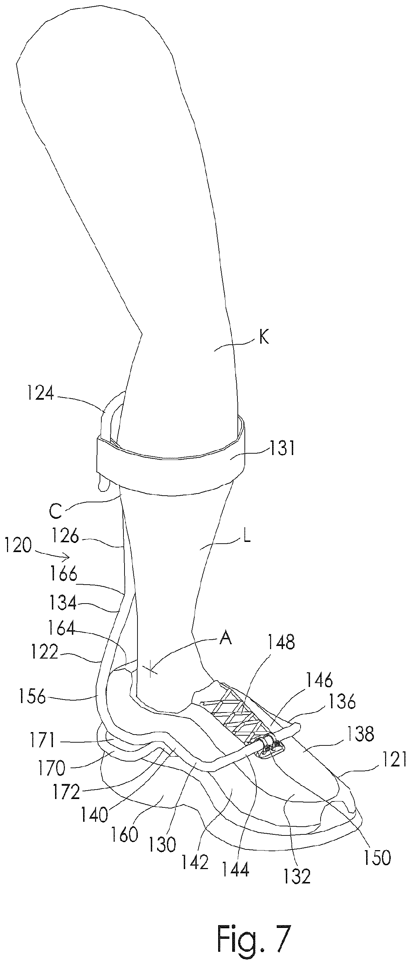

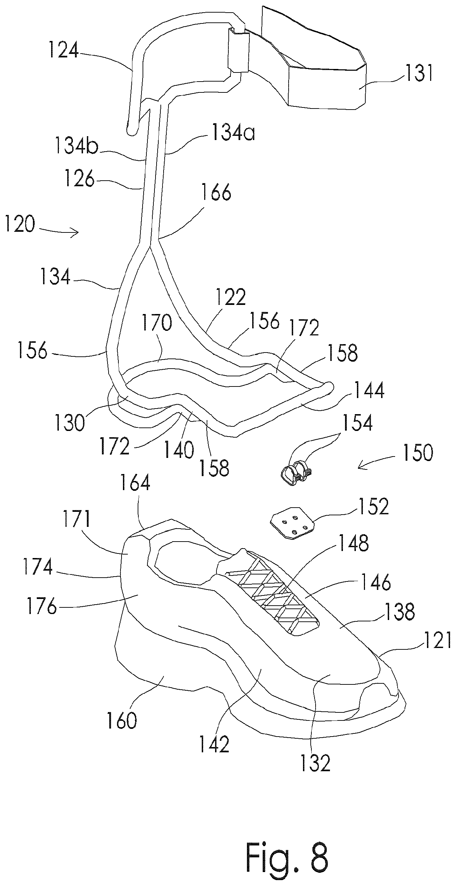

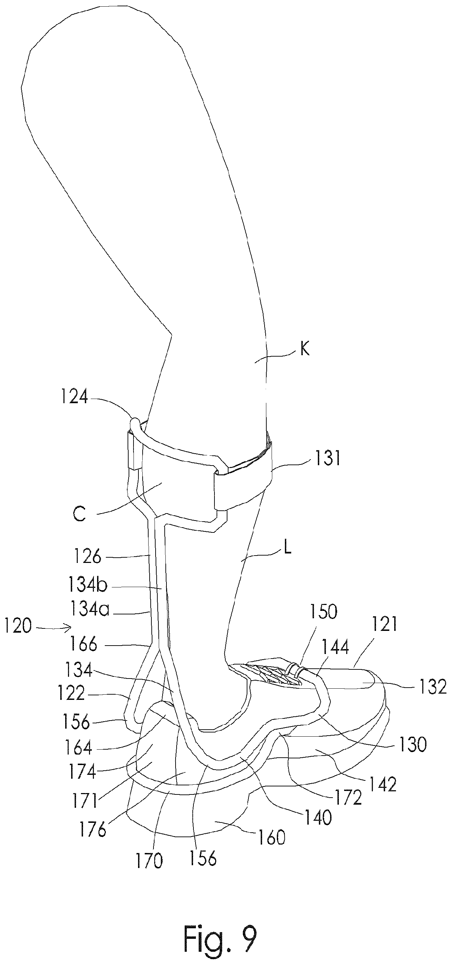

Referring now to Figs. 7 to 9, there is shown another embodiment wherein the features are numbered with reference numerals in the 100 series which correspond to the reference numerals of the previous embodiment. As it will be described in more details below, the orthotic foot brace 120 has a frame 122 similar to the brace 20 shown in Figs. 1 to 6. However, the foot band 62 of the brace 20 is replaced by a heel support 170 extending between the lateral and median sections 136, 140 of the frame 122 and behind the footwear heel 171. As for the above described embodiment, only the left foot brace which is securable to the person's left lower leg is shown and described below.

The lower leg holder 124 including the leg attachment strap 131 will not be described in details since they are similar to the ones described above in reference to Figs. 1 to 6. Furthermore, the vertical and the foot sections 126, 130 of the frame 122 and the attachment means of the frame 122 to the footwear 121 will not be described in detail since they are also similar to the ones described above in reference to Figs. 1 to 6. As for the above-described brace 20, the brace 120 is located entirely outside of the footwear 121.

The heel support 170 is part of the brace frame 122. It has two opposite ends 172 attached to the straight section 158 of the frame member 134 in the lateral and median sections 136, 140, close to the end of the curved shaped section 156. It extends rearwardly of the footwear 121, behind the heel section 171. It is located in the lower portion of the footwear heel 171 above the outsole 60 and in the lower section of the footwear counter 174, i.e. the reinforcement used to maintain the heel of the foot, if any. The heel support 170 applies a compression force on the footwear quarters 176. The compression force maintains the heel support 170 in contact with the footwear 121 and substantially prevents or reduces its displacement relatively to the footwear 121. Thus, the lateral and median portions of the heel support 170 are slightly pulled away to insert to footwear quarters 176 therebetween. When disengaged from the footwear 121, the spacing between the lateral and median portions of the heel support 170 is slightly narrower than the thickness of the footwear quarters 176 where the heel support 170 is engaged.

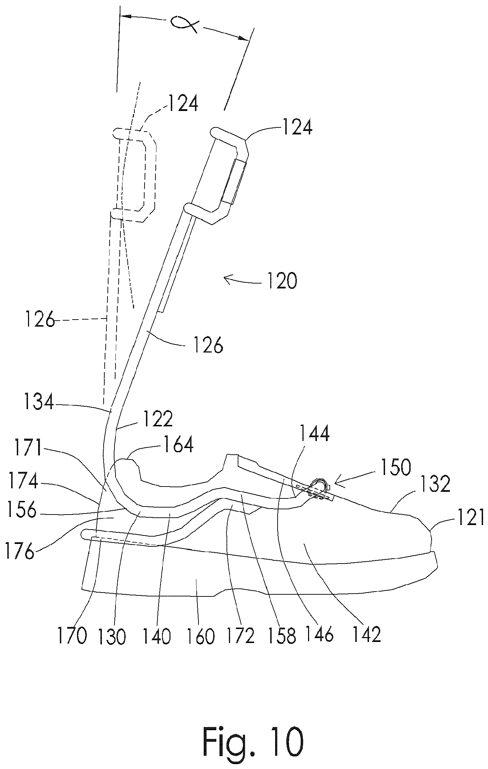

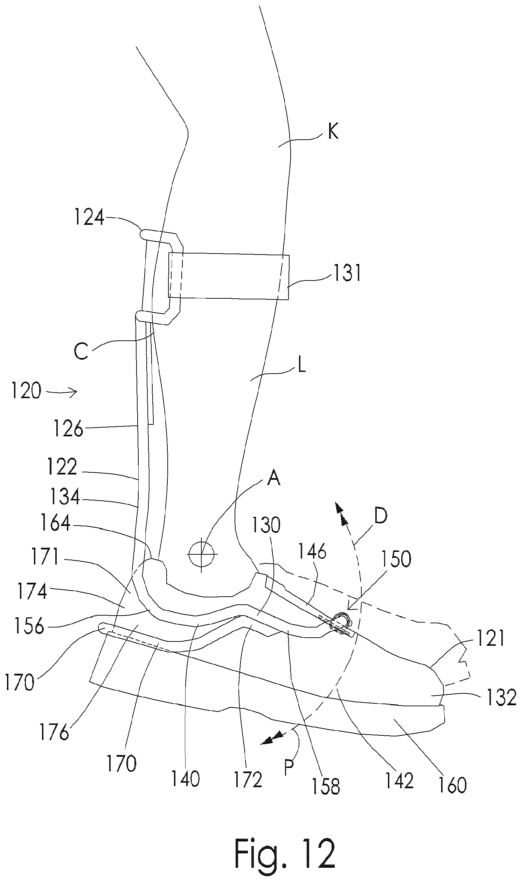

Referring now to Figs. 10 to 12 and 16, there is shown that the heel support 170 can be attached to different sections of the brace frame 122. In Figs. 10 to 12, sections of the heel support 170, close to the ends 172, are attached and juxtaposed to the straight section 158 of the frame member 134 in the lateral and median sections 136, 140. In Fig. 12, sections of the heel support 170, close to the ends 172, are attached and juxtaposed to the end of the curved shaped sections 156 in the lateral and median sections 136, 140. It is appreciated that, in alternative embodiments (not shown), the heel support 170 can be attached to different sections of the brace frame 122. For instance and without being limitative, the heel support 170 can be mounted anywhere along the lateral and median sections 136, 140.

Fig. 10 shows the brace 120 secured to the footwear 121 but without being engaged with a person's lower leg L. As for the above-described brace 20, the vertical strut 126 extends forwardly towards the toe section 132 of the footwear 121. To attach the brace 120 to the lower leg L, the vertical section 126 is pulled rearwardly and, when attached, the lower leg holder 124 and the vertical section 126 apply a forwardly oriented pressure, or a compression force on the lower leg L. In a non-operative configuration, i.e. when detached from the lower leg L, the brace 120 defines an angle ranging between 10 and 30 degrees and, in an alternative embodiment, between 15 and 20 degrees. As mentioned above, the compression stress applied to the person's lower leg L restricts the plantarflexion P of the foot and creates a bias for the dorsiflexion D of the foot.

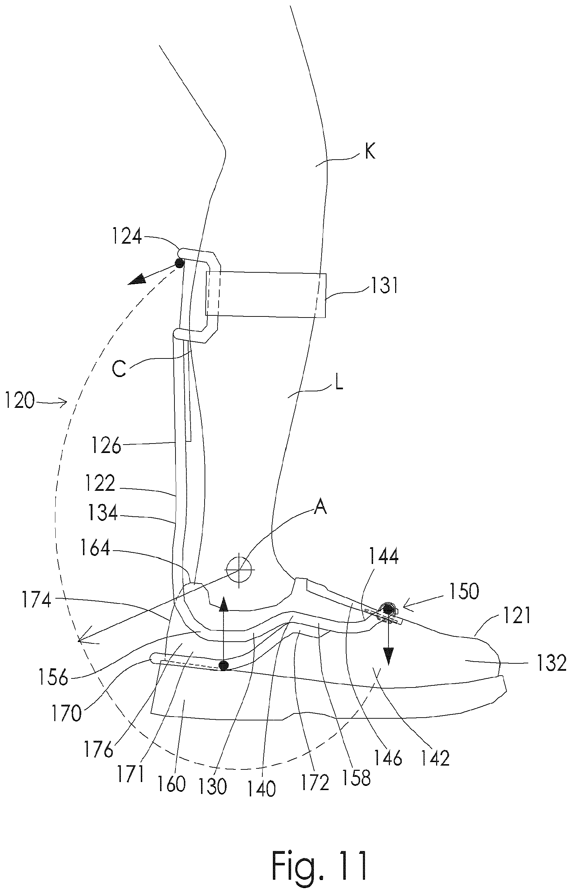

Referring to Figs. 11 and 12 and as mentioned above, there is shown that during gaiting, the foot including the footwear 121 performs plantarflexions P and pivots about a pivoting axis which substantially corresponds to the ankle joint A. As a result, an upwardly oriented force is applied on the heel support 170 when the foot performs plantarflexions P. Since the heel support 170 applies a compression force on the footwear quarters 176 which reduces displacement of the heel support 170 relatively to the footwear 121, the heel support 170 restricts the plantarflexion P of the foot, i.e. it creates a resistance to the plantarflexion moment P. A rearwardly extending tension is also applied on the lower leg holder 124. As mentioned above, the lower leg holder 124 applies a compression force on the lower leg L. Therefore, the combination of the upwardly oriented force on the heel support 170, the rearwardly extending tension and the natural compression force applied on the lower leg L creates the resulting dorsiflexion moment D. The brace 120 conveys the foot including the footwear 121 to return to its normal, resting position.

The heel support 170 acts as a retaining member by restraining a rearwardly and downwardly oriented pivotal movement of the brace 120 and, more particularly, by restraining a downward movement of the lower leg holder 124, of the vertical section 126, and/or of the curved sections 156 during gaiting.

As for the same brace 20, the brace 120 can be used for either the left or the right foot.

In alternative embodiments (not shown), the heel support can be divided in two spaced-apart sections extending rearwardly towards the footwear heel from the lateral and median sections 136, 140, without being connected to one another. Thus, each heel support sections have a rearwardly extending free end.

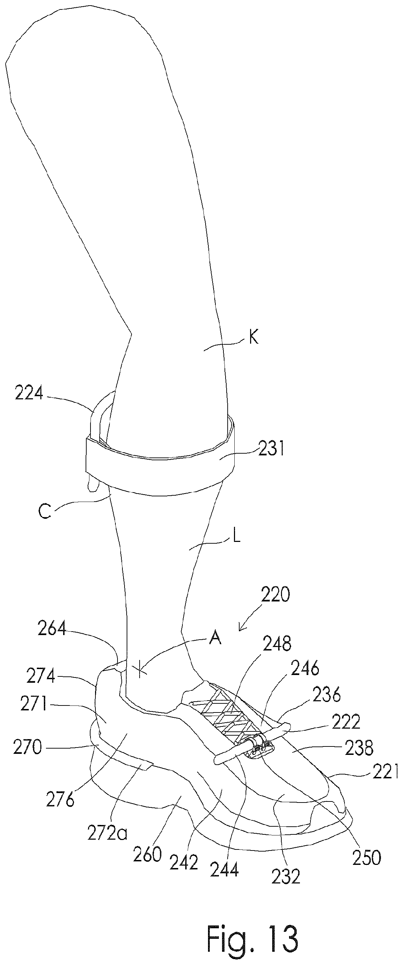

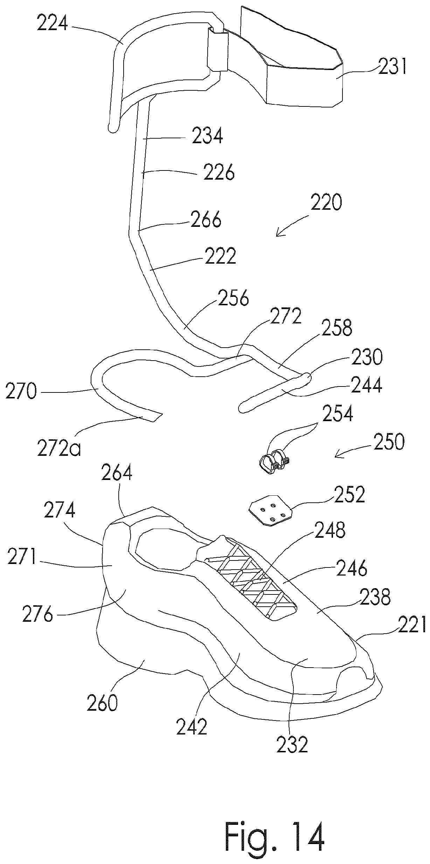

Referring now to Figs. 13 to 17, there is shown another embodiment wherein the features are numbered with reference numerals in the 200 series which correspond to the reference numerals of the previous embodiments. As it will be described in more details below, the frame 222 of the orthotic foot brace 220 differs from the frames 22, 122 of braces 20 and 120 shown in Figs. 1 to 12. The brace 220 does not include a foot band 62 but a heel support 270 extending between the lateral and median sides 238, 242 and behind the footwear heel 271. Furthermore, only one end of the heel support 270 is secured to the foot section 230 of the brace 220. For conciseness, only the left foot brace which is securable to the person's left lower leg is shown and described below. For this embodiment, the right foot brace is a mirror image thereof.

The lower leg holder 224 including the leg attachment strap 231 will not be described in detail since they are similar to the ones described above in reference to Figs. 1 to 6. Furthermore, the attachment means of the frame 222 to the footwear 221 will not be described in detail since they are also similar to the ones described above in reference to Figs. 1 to 6. As for the above-described braces 20, 120, the brace 220 is entirely located outside of the footwear 221, i.e. it is juxtaposed to an outer surface of the footwear 221.

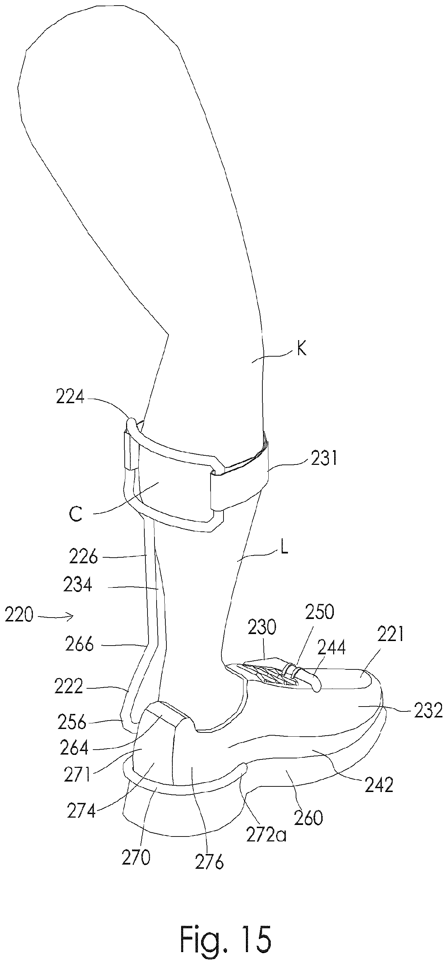

In the vertical section 226, a single rod, or frame member 234, extends downwardly from the lower leg holder 224 towards the foot section 230. On the opposite of the above-described braces 20, 120, the foot section 230 includes a frame member 234 extending continuously from the vertical section 226 to the front section 244 juxtaposed to the lateral side of the footwear 221. No frame member 234 extends continuously from the vertical section 226 to the front section 244 on the median side 242 of the footwear 221. The junction between the distal end 266 of the vertical section 226 and the curved section 256 of the frame member 234 in the foot section 230 is located above the binding 264 of the footwear 221 to allow plantarflexion P, as shown in Fig. 15. The shape of the frame member 234 in the lateral section 236 and the front section 244 as well as the attachment means 250 to the footwear 221 in the front section 244 are similar to the ones of the above-described embodiments. Therefore, they will not be described in further detail.

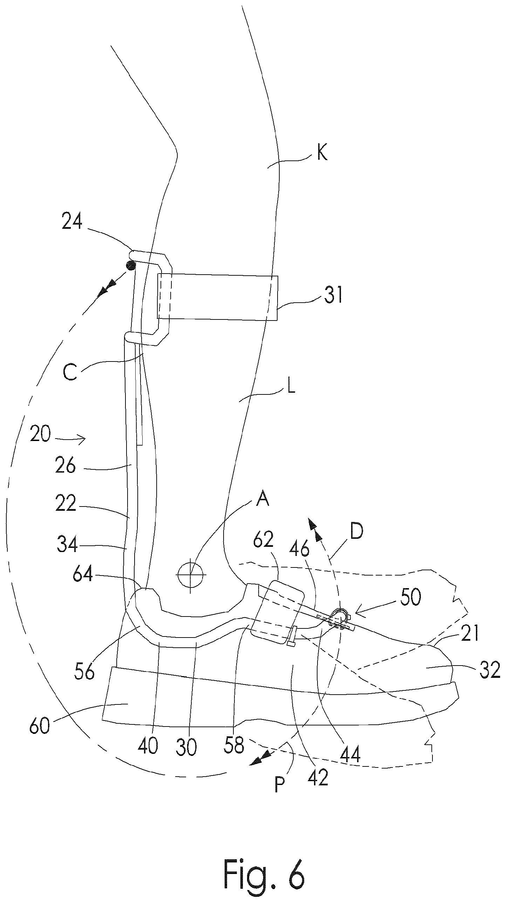

The heel support 270 of the brace 220 has two opposite ends 272, the lateral end 272a is attached to the straight section 258 of the frame member 234 in the lateral section 236, close to the end of the curved shaped section 256. As for the embodiment described in reference to Figs. 6 to 8, the heel support 270 can be attached to a different section of the brace frame 222. On the opposite of brace 120, the median end 272b of the heel support 270 is not attached to the median section 240, i.e. it is unattached. The median end 272b is juxtaposed to the median side 242 of the footwear 221 above the outsole 260, close to an inner arch of the footwear 221. The heel support 270 extends rearwardly of the footwear 221, behind the heel section 271. From the heel section 271 to the median side 242 of the footwear 221, the heel support 270 follows a junction of an upper end of the outsole 260 and a lower end of the footwear quarters 276..

It is appreciated that in an alternative embodiment (not shown), the heel support 270 can be attached to the median section 240 of the brace frame 222 and have an unattached (or free) lateral end 272a. Furthermore, as mentioned for the heel support 270, the heel support 270 can be attached to different sections of the brace frame 222.

As for the above-described heel support 270, the heel support 270 applies a compression force on the footwear quarters 276 which maintains the heel support 270 in contact with the footwear 221 and substantially prevents or reduces its displacement relatively to the footwear 221. The heel support 270 acts as a retaining member by restraining a rearwardly and downwardly oriented pivotal movement of the brace 220 and, more particularly, at restraining a downward movement of the lower leg holder 224, of the vertical section 226, and/or of the curved section 256 during gaiting.

As for the above-described embodiment, when disengaged from the lower leg L, the vertical strut 226 extends forwardly towards the toe section 232 of the footwear 221. Thus, a compression stress is applied to the person's lower leg L when engaged with the brace 220. The compression stress restricts the plantarflexion P of the foot and creates a bias for the dorsiflexion D of the foot.

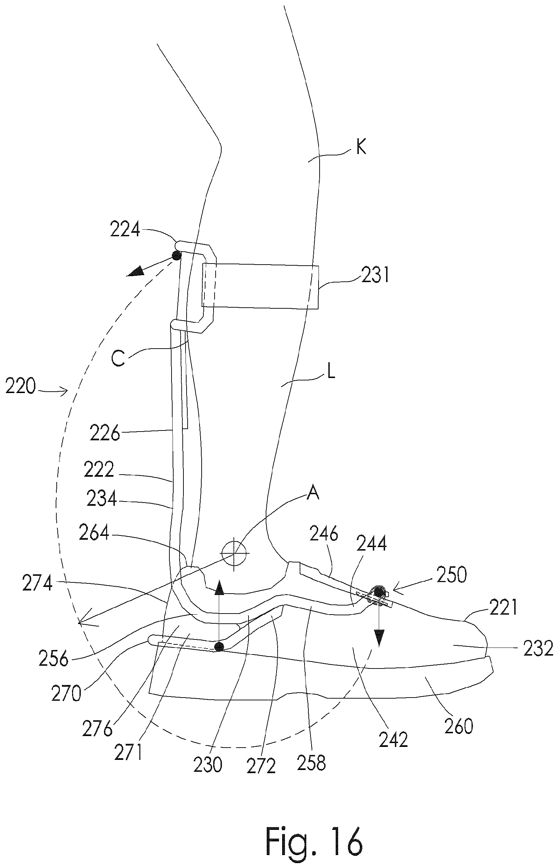

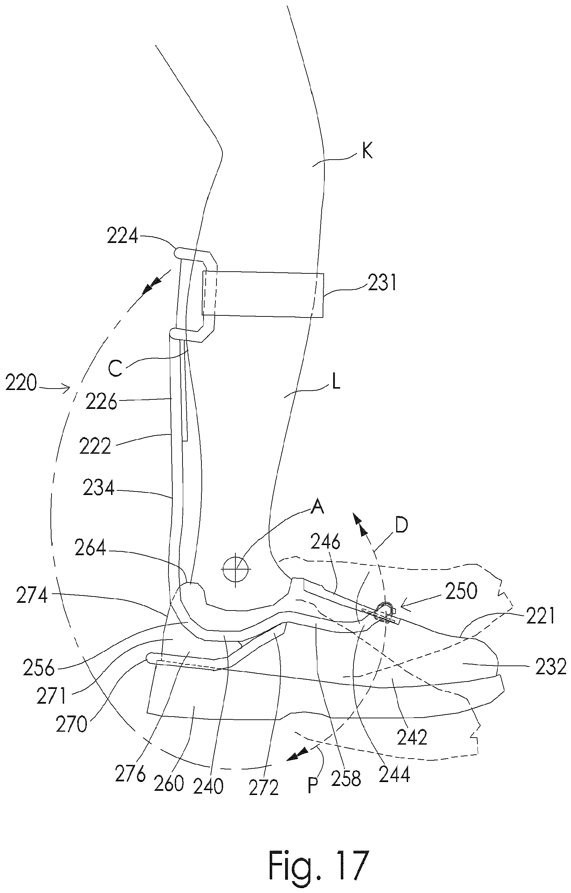

Referring to Figs. 16 and 17, there is shown that during gaiting, the brace 220 performs the same function as the brace 120 described above in reference to Figs. 7 to 12. The heel support 270 applies a compression force on the footwear quarters 276 and restricts the plantarflexion P of the foot. The combination of the upwardly oriented force, of the rearwardly extending tension and the natural compression force applied on the lower leg L conveys the foot including the footwear 221 to return to its normal, resting position during gaiting.

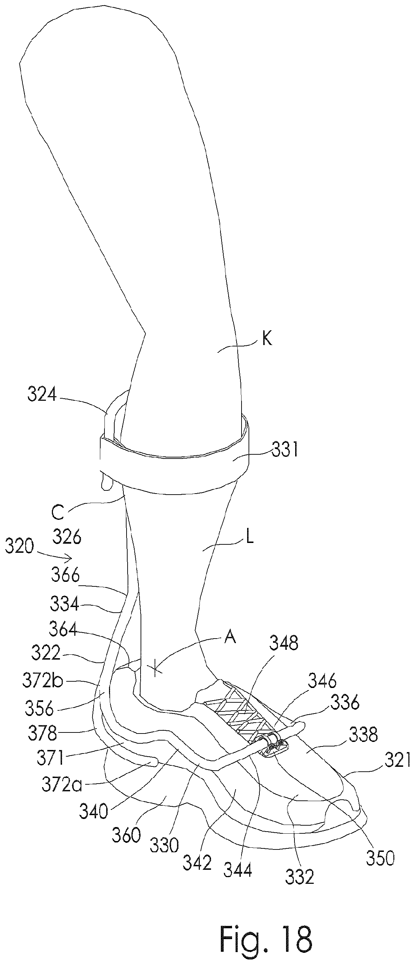

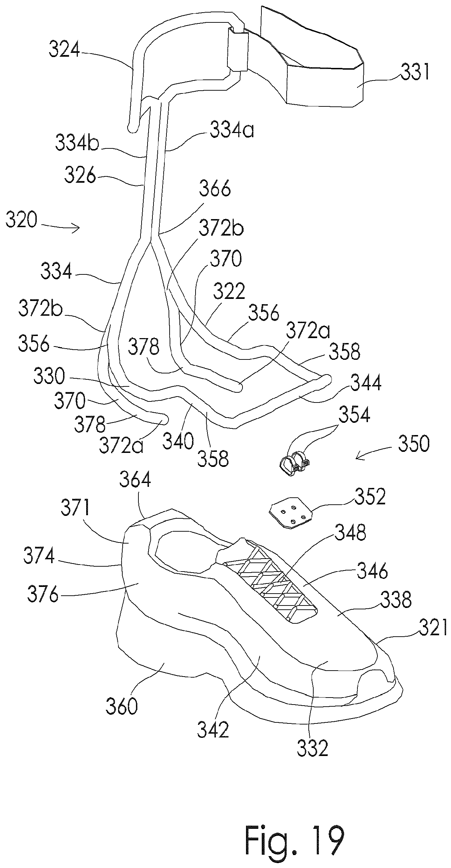

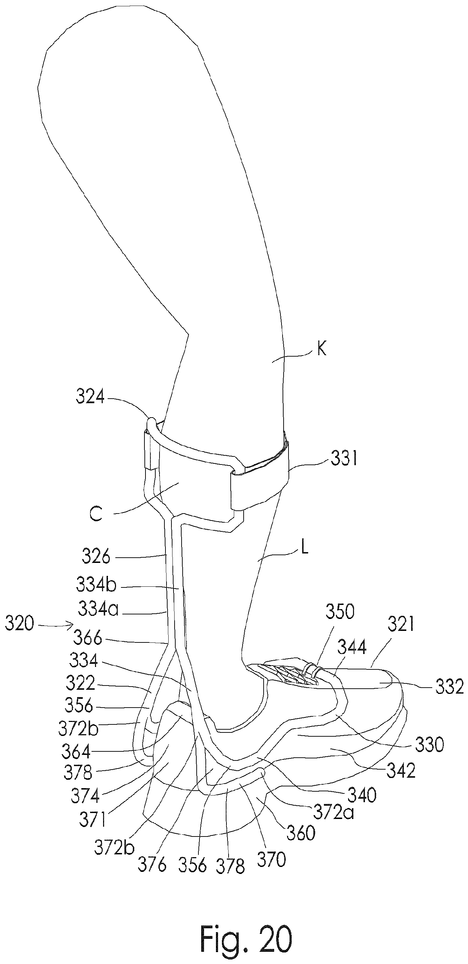

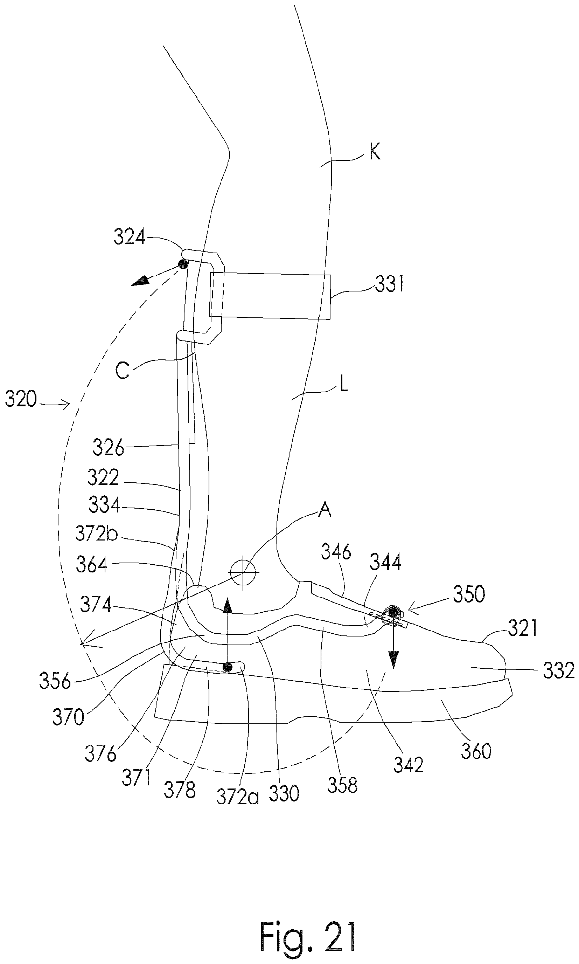

Referring now to Figs. 18 to 21, there is shown another embodiment wherein the features are numbered with reference numerals in the 300 series which correspond to the reference numerals of the previous embodiments. As it will be described in more details below, the frame 322 of the orthotic foot brace 320 differs from the frames 22, 122, 222 of braces 20, 120, 220 shown in Figs. 1 to 17. As braces 120, 220, the brace 320 includes a heel support 370 having two spaced-apart heel support sections 378 and, more particularly, a median heel support section extending on the median side 342 of the footwear 321 and a lateral heel support section extending on the lateral side 338 of the footwear 321. Each heel support section 378 extends from the curved shaped section 356 of the frame 322 towards the toe section 332. A first end 372b of each one of the heel support sections 378 is connected/secured to the foot section 330 of the brace 320 and the second end 372a of each one of the heel support section 378, opposed to the first end 372b, is free and is juxtaposed to either the lateral or median sides 338, 342 of the footwear 321. The free ends 372a are located close to the inner arches of the footwear 321, above the outsole 360. A section of each one of the heel support sections 378, extending from the free end 372a towards the opposed end 372b, follows a junction of an upper end of the outsole 360 and a lower end of the footwear quarters 376.

As for the above-described heel supports 170, 270, the heel support sections 378 apply a compression force on the footwear quarters 376 which maintains the heel support 370 in contact with the footwear 321 and substantially prevents or reduces its displacement relatively to the footwear 321. The heel support 370 acts as a retaining member by restraining a rearwardly and downwardly oriented pivotal movement of the brace 320 and, more particularly, at restraining a downward movement of the lower leg holder 324, of the vertical section 326, and/or of the curved shaped section 356 during gaiting.

Referring to Figs. 16 and 17, there is shown that during gaiting, the brace 320 performs the same function as braces 120, 220 described above in reference to Figs. 7 to 17. Furthermore, when disengaged from the lower leg L, the vertical strut 326 extends forwardly towards the toe section 332 of the footwear 321. Thus, a compression stress is applied to the person's lower leg L when engaged with the brace 320 to restrict the plantarflexion P of the foot and create a bias for the dorsiflexion D of the foot.

As for the above-described braces 20, 120, 220, the brace 320 is entirely located outside of the footwear 321, i.e. it is juxtaposed to an outer surface of the footwear 321. For conciseness, only the left foot brace which is securable to the person's left lower leg is shown and described below. For this embodiment, the right foot brace is a mirror image thereof.

In the embodiments shown, the heel support is attached to the lateral and median sections of the frame member. However, in alternative embodiments, the heel support and the lateral and median sections of the frame member can be single piece.

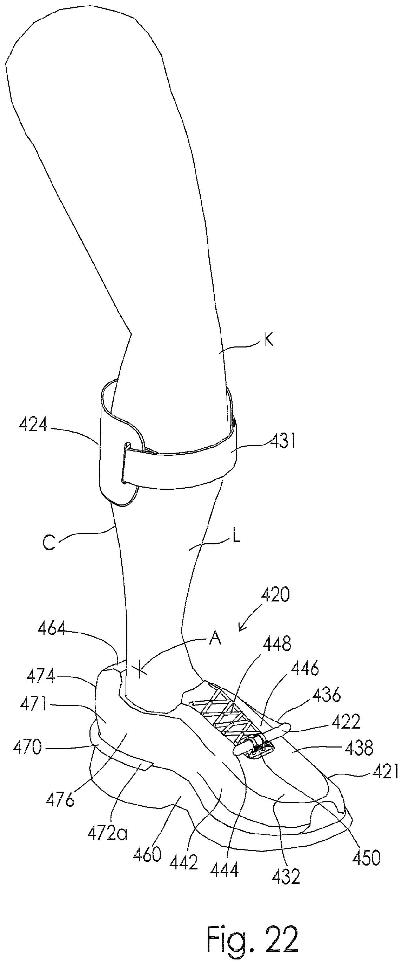

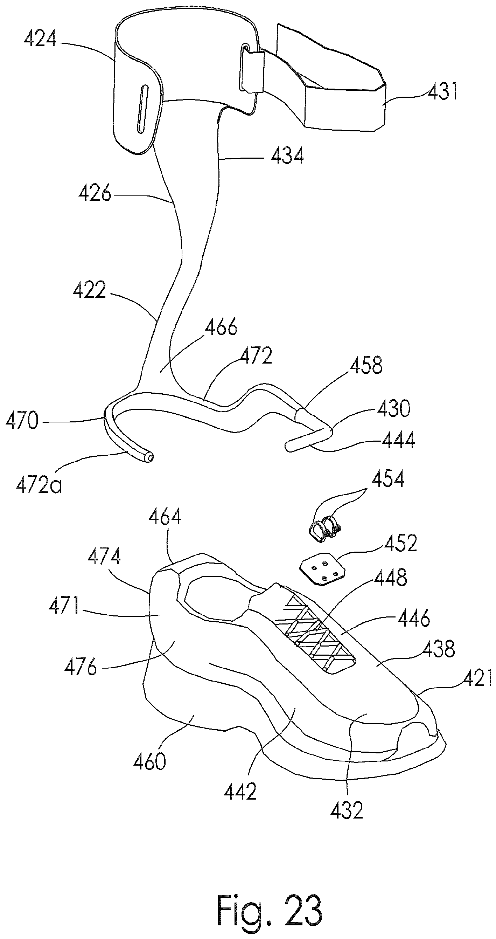

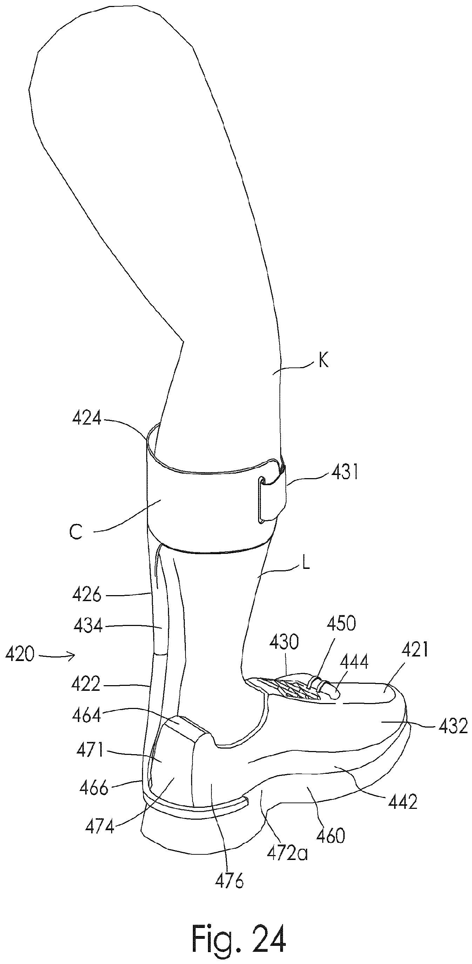

Referring now to Figs. 22 to 25, there is shown another embodiment wherein the features are numbered with reference numerals in the 400 series which correspond to the reference numerals of the previous embodiments. As it will be described in more details below, the frame 422 of the orthotic foot brace 420 differs from the above-described frames of braces 20, 120, 220, 320 shown in Figs. 1 to 21. As the braces 120, 220, 320, the brace 420 includes a heel support 470 extending between the lateral and median sides 438, 442 of the footwear and behind the footwear heel 471. It surrounds (contours) the footwear quarters 476 of the footwear 421. Furthermore, only one end of the heel support 470 is secured to the foot section 430 of the brace 420. For conciseness, only the left foot brace which is securable to the person's left lower leg is shown and described below. For this embodiment, the right foot brace is a mirror image thereof.

The lower leg holder 424 including the leg attachment strap 431 will not be described in detail since they are similar to the ones described above in reference to Figs. 1 to 6. Furthermore, the attachment means of the frame 422 to the footwear 421 will not be described in detail since they are also similar to the ones described above in reference to Figs. 1 to 6. As for the above-described braces 20, 120, 220, 320, the brace 420 is entirely located outside of the footwear 421, i.e. it is juxtaposed to an outer surface of the footwear 421.

In the vertical section 426, a single rod, or frame member 434, extends downwardly from the lower leg holder 424 towards the foot section 430 and the heel support 470. More particularly, the lower leg strut 426 joins and is connected to the heel support 470 at a lower and distal end 466 thereof.

The foot section 430 and the heel support 470 are single piece and extend continuously, i.e. the juxtaposed ends of the foot section 430 and the heel support 470 is definite. The foot section 430 is juxtaposable to the lateral side 438 of the footwear 421. The vertical section 426 extends upwardly from the heel support 470, close to the junction of the foot section 430 and the heel support 470.

As mentioned above, the heel support 470 of the brace 420 has a first end which connects continuously with the foot section 430 on the lateral side 438 of the footwear 421. The other and opposed end of the heel support 470, i.e. the median end 472a, of the heel support 470, is not attached to the foot section 430, i.e. it is unattached. The median end 472a is juxtaposed to the median side 442 of the footwear 421 above the outsole 460, close to an inner arch of the footwear 421.

The heel support 470 extends rearwardly of the footwear 421, behind the heel section 471. From the heel section 471 to the median side 442 of the footwear 421, the heel support 470 substantially follows a junction of an upper end of the outsole 460 and a lower end of the footwear quarters 476.

It is appreciated that, in an alternative embodiment (not shown), the foot section can extend on the median side 442 of the footwear 421 and the heel support 470 can be attached to the median section 440 of the brace frame 422 and have an unattached (or free) lateral end.

The heel support 470 applies a compression force on the footwear quarters 476 which maintains the heel support 470 in contact with the footwear 421 and substantially prevents or reduces its displacement relatively to the footwear 421. The heel support 470 acts as a retaining member by restraining a rearwardly and downwardly oriented pivotal movement of the brace 420 and, more particularly, at restraining a downward movement of the lower leg holder 424 and/or the vertical section 426 (or lower leg strut) during gaiting.

As mentioned above, the heel support 470 applies a compression force on the footwear but is unattached or unfastened thereto.

As for the above-described embodiment, when disengaged from the lower leg L, the vertical strut 426 extends forwardly towards the toe section 432 of the footwear 421. Thus, a compression stress is applied to the person's lower leg L when engaged with the brace 420. The compression stress restricts the plantarflexion P of the foot and creates a bias for the dorsiflexion D of the foot.

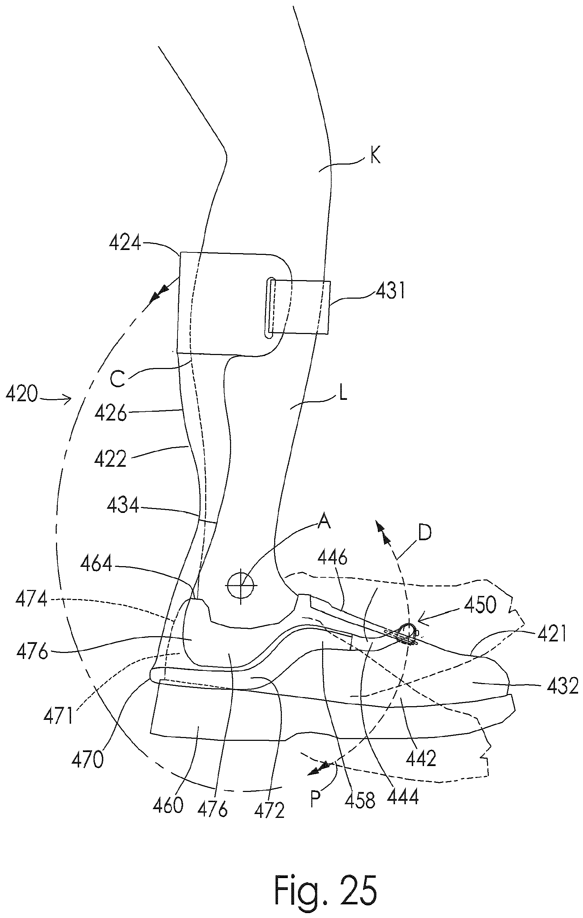

Referring to Fig. 25, there is shown that during gaiting, the heel support 470 applies a compression force on the footwear quarters 476 and restricts the plantarflexion P of the foot. The combination of the upwardly oriented force, of the rearwardly extending tension and the natural compression force applied on the lower leg L conveys the foot including the footwear 421 to return to its normal, resting position during gaiting.

As mentioned above, the heel support of the brace has a first end which connects continuously with the foot section on the lateral side of the footwear. The other and opposed end of the heel support, i.e. the median end, of the heel support, is not attached to the foot section, i.e. it is unattached. The median end is juxtaposed to the median side of the footwear above the outsole, close to an inner arch of the footwear.

In an alternative embodiment (not shown), the foot section can include a lateral section juxtaposable to the lateral side of the footwear and a median section juxtaposable to the median side of the footwear and connecting in the front section. The brace also includes a heel support that extends continuously and rearwardly from the lateral and median sections of the foot section and contours the quarters of the footwear. Thus both ends of the heel support are connected to a respective one of the lateral and median sections of the foot section. The same brace can be used either for the left foot or the right foot.

Alternative embodiments can be foreseen to the above-described braces. For instance and without being limitative, the braces can include two spaced-apart vertical struts connected to the lower leg holder at a first end and connected either to the heel support, if any, or the foot section.

The braces 20, 120, 220, 320, 420 are located outwardly of the footwear 21, 121, 221, 321, 421 and are juxtaposed to the footwear outer surface. Thus, both shoes can be of the same size and no friction during gaiting is applied directly to the person's foot. It is adapted to fit on most conventional shoes. It is appreciated that the attachment means can be adapted to fit on any appropriate shoe. Furthermore, the braces 20, 120, 220, 320, 420 are adapted for sports such as running.

The brace frame can be made of several materials. For instance and without being limitative, it can be made of rigid, strong, relatively light-weight polymer materials such as thermoplastic or thermosetting polymer, plastic, fiber reinforced plastic, molded chopped fibers, laminates or any other suitable material. Other suitable materials can include metals and alloys. Exemplary materials include, but are not limited to, nylons, glass filled nylon, polypropylenes, vinyls, polyvinyl chlorides, high density polyethylene, epoxies, urethanes, and polyesters. Carbon/graphite fiber materials can also be used because of their relatively high strength and their relatively low weight.

As mentioned above, the shape of the frame member can differ from the one shown in the above described embodiments. The cross-sectional shape of the frame member can be substantially flat or any other appropriate shape instead of being circular. It can be also be thicker, narrower, larger, etc. It can be discontinuous, i.e. it can include several juxtaposed and attached components. The frame member can be one single piece in the vertical section instead of two juxtaposed frame member sections.

The braces can be easily detached from the person's lower leg and footwear and remove. Further, since the brace is juxtaposed to the outer surface of the footwear and is not inserted in the inner space of the footwear, foot wounds are prevented.

In an alternative embodiment, the brace length, either in the vertical section or in the foot section, can be adjustable. For instance and without being limitative, the frame can include sliding components such as two frame member slidingly attached to one another.

The frame components such as the heel support can include anti-slip coating or features to further restrain the rearwardly and downwardly oriented pivotal movement of the brace. The brace and the footwear can included complementary Velcro members as anti-slip features. The brace can include adhesive coatings. It can also include nails or screws for securing at least sections thereof to the footwear. Furthermore, sections of the frame can include compressible material such as foam or neoprene to facilitate fitting to a person's lower leg and footwear.

Several alternative embodiments and examples have been described and illustrated herein. The embodiments of the invention described above are intended to be exemplary only. A person of ordinary skill in the art would appreciate the features of the individual embodiments, and the possible combinations and variations of the components. A person of ordinary skill in the art would further appreciate that any of the embodiments could be provided in any combination with the other embodiments disclosed herein. It is understood that the invention may be embodied in other specific forms without departing from the central characteristics thereof. The present examples and embodiments, therefore, are to be considered in all respects as illustrative and not restrictive, and the invention is not to be limited to the details given herein. Accordingly, while the specific embodiments have been illustrated and described, numerous modifications come to mind without significantly departing from the scope the invention. The scope of the invention is therefore intended to be limited solely by the scope of the appended claims.

## Claims

1. An orthotic foot brace (120, 220, 320, 420) for a person wearing a footwear comprising: a lower leg holder (124) securable around a lower leg of the person;
a lower leg strut (126) secured to the lower leg holder and extending downwardly towards the footwear;
a foot strut connected to the lower leg strut (126) and having at least one of a median section (140) and a lateral section (136) juxtaposable to a respective side of the footwear and outwardly thereof, and a front section (144) securable to an instep section (146) of the footwear;
and
at least one brace retaining member connected to the foot strut, juxtaposable to the footwear, and having at least a section extending on the median side of the footwear and at least a section extending on the lateral side of the footwear, outwardly thereof, and restraining at least one of a rearwardly and downwardly oriented pivotal movement of the brace, the at least one brace retaining member having a heel support (170) extending rearwardly of the footwear and being connected to at least one of the lateral section and the median section of the foot strut.

2. An orthotic foot brace as claimed in claim 1, wherein the at least one brace retaining member extends continuously from the median side of the footwear to the lateral side of the footwear.

3. An orthotic foot brace as claimed in any one of claims 1 to 4, wherein the lower leg strut (126) has a distal end and the foot strut comprises said lateral section and said median section (140), spaced- apart from the lateral section (136) and connecting at the distal end of the lower leg strut.

4. An orthotic foot brace as claimed in claim 1, wherein the heel support (170) contours quarters (176) of the footwear and is configured to extend above an outsole (60) of the footwear, the heel support being connected to the lower leg strut.

5. An orthotic foot brace as claimed in claim 4, wherein the foot strut comprises said lateral section (136) extending on a lateral side of the footwear and said median section (140) extending on a median side of the footwear and the heel support (170) extends rearwardly from the lateral section (136) extending on the lateral side of the footwear and the median section (140) extending on the median side of the footwear.

6. An orthotic foot brace as claimed in claim 4, wherein the heel support (170) extends rearwardly from the one of the lateral section (136) extending on the lateral side of the footwear and the median section (140) extending on the median side of the footwear.

7. An orthotic foot brace as claimed in one of claims 4 and 6, wherein the foot strut comprises said lateral section (136) and the heel support (170) is connected to the foot strut in the lateral section.

8. An orthotic foot brace as claimed in claim 4 to 7, wherein the heel support (170) extends rearwardly and continuously from the foot strut.

9. An orthotic foot brace (220) as claimed in one of claims 4 and 6, wherein the heel support (270) comprises an unattached end (272a) juxtaposable to one of the median side and the lateral side of the footwear,
and wherein the lower leg strut (226) extends upwardly from one of the heel support and the foot strut,
and wherein the heel support compresses the footwear quarters when engaged with the footwear,
and wherein the heel support and the foot strut being single piece,
or wherein the lower leg strut, the foot strut, and the heel support being single piece.

10. An orthotic foot brace as claimed in any one of claims 1 to 9, wherein the lower leg strut (126) applies a forwardly oriented pressure on the lower leg when engaged therewith.

11. An orthotic foot brace (320) as claimed in claim 1, wherein the heel support comprises a median heel support section (378) extending on the median side of the footwear and extending forwardly from the median section of the brace juxtaposed to a heel section of the footwear and the heel support further comprises a lateral heel support section (378) extending on the lateral side of the footwear juxtaposed to the heel section of the footwear and extending forwardly from the lateral section of the brace, each one of the median and lateral heel support sections having an unattached end (372a), each unattached ends of the median and lateral heel support sections extending towards a toe section of the footwear.

12. An orthotic foot brace (320) as claimed in any one of claims 1 to 11, further comprising a second lower leg strut extending upwardly from one of a heel support and the foot strut and spaced-apart from the first lower leg strut.

## Patentansprüche

1. Orthopädische Fußstütze (120, 220, 320, 420) für eine Person, die ein Schuhwerk trägt, umfassend: einen Unterschenkelhalter (124), der um einen Unterschenkel der Person befestigt werden kann;
eine Unterschenkelstrebe (126), die an dem Unterschenkelhalter befestigt ist und sich nach unten zu dem Schuhwerk hin erstreckt; eine Fußstrebe, die mit der Unterschenkelstrebe (126) verbunden ist und mindestens einen von einem Mittelabschnitt (140) und einem Seitenabschnitt (136) aufweist, die angrenzend an einer jeweiligen Seite des Schuhwerks und äußerlich davon angeordnet sind, und einen Vorderabschnitt (144) aufweist, der an einem Ristabschnitt (146) des Schuhwerks befestigt werden kann;
und
mindestens ein Stützenhalteelement, das mit der Fußstrebe verbunden ist und an das Schuhwerk angrenzend angeordnet werden kann, und mindestens einen Abschnitt aufweist, der sich auf der Mittelseite des Schuhwerks erstreckt, und mindestens einen Abschnitt aufweist, der sich auf der Lateralseite des Schuhwerks äußerlich davon erstreckt, und mindestens eine von einer nach hinten gerichteten und einer nach unten gerichteten Schwenkbewegung der Strebe zurückhält, wobei das mindestens eine Stützenhalteelement eine Fersenstütze (170) aufweist, die sich rückseitig des Schuhwerks erstreckt und mit mindestens einem von dem Seitenabschnitt und dem Mittelabschnitt der Fußstrebe verbunden ist.

2. Orthopädische Fußstütze nach Anspruch 1, wobei sich das mindestens eine Stützenhalteelement kontinuierlich von der Mittelseite des Schuhwerks zur Lateralseite des Schuhwerks erstreckt.

3. Orthopädische Fußstütze nach einem der Ansprüche 1 bis 4, wobei die Unterschenkelstrebe (126) ein distales Ende aufweist und die Fußstrebe den Seitenabschnitt und den Mittelabschnitt (140), der von dem Seitenabschnitt (136) beabstandet ist, umfasst und am distalen Ende der Unterschenkelstrebe mit dieser verbunden ist.

4. Orthopädische Fußstütze nach Anspruch 1, wobei die Fersenstütze (170) Schafthinterteile (176) des Schuhwerks nachformt und so ausgestaltet ist, dass sie sich oberhalb einer Laufsohle (60) des Schuhwerks erstreckt, wobei die Fersenstütze mit der Unterschenkelstrebe verbunden ist.

5. Orthopädische Fußstütze nach Anspruch 4, wobei die Fußstrebe den Seitenabschnitt (136), der sich auf einer Lateralseite des Schuhwerks erstreckt, und den Mittelabschnitt (140), der sich auf einer Mittelseite des Schuhwerks erstreckt, umfasst, und sich die Fersenstütze (170) von dem sich auf der Lateralseite des Schuhwerks erstreckenden Seitenabschnitt (136) nach hinten erstreckt und sich der Mittelabschnitt (140) auf der Mittelseite des Schuhwerks erstreckt.

6. Orthopädische Fußstütze nach Anspruch 4, wobei sich die Fersenstütze (170) von dem einen von dem Seitenabschnitts (136), der sich auf der Lateralseite des Schuhwerks erstreckt, und dem Mittelabschnitt (140), der sich auf der Mittelseite des Schuhwerks erstreckt, nach hinten erstreckt.

7. Orthopädische Fußstütze nach einem der Ansprüche 4 und 6, wobei die Fußstrebe den Seitenabschnitt (136) umfasst und die Fersenstütze (170) mit der Fußstrebe in dem Seitenabschnitt verbunden ist.

8. Orthopädische Fußstütze nach Anspruch 4 bis 7, wobei sich die Fersenstütze (170) nach hinten und kontinuierlich von der Fußstrebe aus erstreckt.

9. Orthopädische Fußstütze (220) nach einem der Ansprüche 4 und 6, wobei die Fersenstütze (270) ein unbefestigtes Ende (272a) umfasst, das an eine von der Mittelseite und der Lateralseite des Schuhwerks angrenzend angeordnet werden kann,
und wobei sich die Unterschenkelstrebe (226) von einer von der Fersenstütze und der Fußstrebe nach oben erstreckt,
und wobei die Fersenstütze die Schuhschafthinterteile zusammendrückt, wenn sie mit dem Schuhwerk in Eingriff steht, und wobei die Fersenstütze und die Fußstrebe einteilig sind, oder wobei die Unterschenkelstrebe, die Fußstrebe und die Fersenstütze einteilig sind.

10. Orthopädische Fußstütze nach einem der Ansprüche 1 bis 9, wobei die Unterschenkelstrebe (126) einen nach vorne gerichteten Druck auf den Unterschenkel ausübt, wenn sie mit diesem in Eingriff steht.

11. Orthopädische Fußstütze (320) nach Anspruch 1, wobei die Fersenstütze einen Mittel-Fersenstützabschnitt (378) umfasst, der sich auf der Mittelseite des Schuhwerks erstreckt und sich von dem Mittelabschnitt der Stütze nach vorne an einen Fersenabschnitt des Schuhwerks angrenzend erstreckt, und die Fersenstütze ferner einen seitlichen Fersenstützabschnitt (378) umfasst, der sich auf der Lateralseite des Schuhwerks an den Fersenabschnitt des Schuhwerks angrenzend erstreckt und sich von dem Seitenabschnitt der Stütze nach vorne erstreckt, wobei jeder von dem Mittel- und Seiten-Fersenstützabschnitt ein unbefestigtes Ende (372a) aufweist,
wobei sich die unbefestigten Enden des Mittel- und des Seiten-Fersenstützabschnitts jeweils zu einem Zehenabschnitt des Schuhwerks erstrecken.

12. Orthopädische Fußstütze (320) nach einem der Ansprüche 1 bis 11, ferner umfassend eine zweite Unterschenkelstrebe, die sich von einer der Fersenstütze und der Fußstrebe nach oben erstreckt und von der ersten Unterschenkelstrebe beabstandet ist.

## Revendications

1. Orthèse de pied (120, 220, 320, 420) pour une personne portant une chaussure, comprenant :
un organe de maintien de jambe (124) apte à être fixé autour d'une jambe de la personne ;
un support de jambe (126) fixé à l'organe de maintien de jambe et s'étendant vers le bas en direction de la chaussure ;
un support de pied relié au support de jambe (126) et ayant au moins une parmi une section médiane (140) et une section latérale (136) qui est apte à être juxtaposée à un côté respectif de la chaussure et extérieurement à celle-ci, et une section avant (144) apte à être fixée à une section cou-de-pied (146) de la chaussure ; et
au moins un élément de retenue d'orthèse relié au support de pied, apte à être juxtaposé à la chaussure, et ayant au moins une section s'étendant sur le côté médian de la chaussure et au moins une section s'étendant sur le côté latéral de la chaussure, extérieurement à celle-ci, et limitant au moins un parmi un mouvement de pivotement orienté vers le bas et vers l'arrière de l'orthèse, l'au moins un élément de retenue d'orthèse ayant un support de talon (170) s'étendant en arrière de la chaussure et relié à au moins une parmi la section latérale et la section médiane du support de pied.

2. Orthèse de pied selon la revendication 1, dans laquelle l'au moins un élément de retenue d'orthèse s'étend de manière continue du côté médian de la chaussure au côté latéral de la chaussure.

3. Orthèse de pied selon l'une quelconque des revendications 1 à 4, dans laquelle le support de jambe (126) a une extrémité distale et le support de pied comprend ladite section latérale et ladite section médiane (140), espacée de la section latérale (136) et reliée au niveau de l'extrémité distale du support de jambe.

4. Orthèse de pied selon la revendication 1, dans laquelle le support de talon (170) suit le contour de quartiers (176) de la chaussure et est configuré pour s'étendre au-dessus d'une semelle extérieure (60) de la chaussure, le support de talon étant relié au support de jambe.

5. Orthèse de pied selon la revendication 4, dans laquelle le support de pied comprend ladite section latérale (136) s'étendant sur un côté latéral de la chaussure et ladite section médiane (140) s'étendant sur un côté médian de la chaussure, et le support de talon (170) s'étend vers l'arrière à partir de la section latérale (136) s'étendant sur le côté latéral de la chaussure et de la section médiane (140) s'étendant sur le côté médian de la chaussure.

6. Orthèse de pied selon la revendication 4, dans laquelle le support de talon (170) s'étend vers l'arrière à partir de l'une parmi la section latérale (136) s'étendant sur le côté latéral de la chaussure et la section médiane (140) s'étendant sur le côté médian de la chaussure.

7. Orthèse de pied selon l'une des revendications 4 et 6, dans laquelle le support de pied comprend ladite section latérale (136) et le support de talon (170) est relié au support de pied dans la section latérale.

8. Orthèse de pied selon les revendications 4 à 7, dans laquelle le support de talon (170) s'étend vers l'arrière et de manière continue à partir du support de pied.

9. Orthèse de pied (220) selon l'une des revendications 4 et 6, dans laquelle le support de talon (270) comprend une extrémité non attachée (272a) apte à être juxtaposée à l'un parmi le côté médian et le côté latéral de la chaussure, et
le support de jambe (226) s'étend vers le haut à partir de l'un parmi le support de talon et le support de pied, et
le support de talon comprime les quartiers de chaussure lorsqu'il est engagé à la chaussure, et
le support de talon et le support de pied sont une seule pièce, ou
le support de jambe, le support de pied et le support de talon sont une seule pièce.

10. Orthèse de pied selon l'une quelconque des revendications 1 à 9, dans laquelle le support de jambe (126) applique une pression orientée vers l'avant sur la jambe lorsqu'il est engagé à celle-ci.

11. Orthèse de pied (320) selon la revendication 1, dans laquelle le support de talon comprend une section de support de talon médiane (378) s'étendant sur le côté médian de la chaussure et s'étendant vers l'avant à partir de la section médiane de l'orthèse, juxtaposée à une section de talon de la chaussure, et le support de talon comprend en outre une section de support de talon latérale (378) s'étendant sur le côté latéral de la chaussure, juxtaposée à la section de talon de la chaussure et s'étendant vers l'avant à partir de la section latérale de l'orthèse, chacune des sections de support de talon médiane et latérale ayant une extrémité non attachée (372a), chaque extrémité non attachée des sections de support de talon médiane et latérale s'étendant vers une section orteils de la chaussure.

12. Orthèse de pied (320) selon l'une quelconque des revendications 1 à 11, comprenant en outre un second support de jambe s'étendant vers le haut à partir de l'un parmi un support de talon et le support de pied et espacé du premier support de jambe.
